Europäisches Patentamt

(19) European Patent Office

Office européen des brevets

(11) Publication number: **0 125 833**
**B1**

(12) **EUROPEAN PATENT SPECIFICATION**

(45) Date of publication of patent specification: 02.01.91

(51) Int. Cl.⁵: **C 07 C 29/76,** C 07 C 31/04, C 07 C 7/12, C 07 C 11/09, C 07 C 41/06, C 07 C 43/04

(21) Application number: 84302939.8

(22) Date of filing: 01.05.84

(54) Removal of impurities from hydrocarbons and alcohols.

(30) Priority: 02.05.83 US 490291

(43) Date of publication of application:
21.11.84 Bulletin 84/47

(45) Publication of the grant of the patent:
02.01.91 Bulletin 91/01

(84) Designated Contracting States:
AT BE CH DE FR GB IT LI LU NL SE

(56) References cited:
EP-A-00 479 06
GB-A-2 075 017

Ullmans Enzyklopädie der technischen Chemie
(1977), 13, p. 297
Encyclopedia of polymer Science and
Technology, 1967, 7, p. 719-721

The file contains technical information
submitted after the application was filed and
not included in this specification

(73) Proprietor: ROHM AND HAAS FRANCE, S.A.
La Tour de Lyon 185 Rue de Bercy
F-75579 Paris, Cedex 12 (FR)

(72) Inventor: Rothschild, Walter G.
521 Mentor Avenue
Painesville Ohio 44077 (US)
Inventor: Gilligan III, Thomas J.
11740 Jason Avenue
Painesville Ohio 44077 (US)

(74) Representative: Tanner, James Percival et al
ROHM AND HAAS (UK) LTD. European
Operations Patent Department Lennig House
2 Mason's Avenue
Croydon, Surrey, CR9 3NB (GB)

# EP 0 125 833 B1

**Description**

This invention relates generally to a method for the removal of cationic and ammoniacal impurities from feed streams comprising olefin-containing hydrocarbons and alcohols using an acid cation exchange resin.

An extremely large number of chemical reactions utilize alcohols and hydrocarbons as starting materials. In many of these reactions the purity of the starting materials is critical to the production of acceptable products. The presence of cationic impurities or basic impurities, such as ammonia, has been particularly troublesome.

Traditionally, the removal of cationic and basic impurities from hydrocarbons has been accomplished by acid and water washing (see, for example, EP-A-0,047,906) or by treatment with strong acid ion exchange resins. Purification of alcohols has also been carried out with strong acid ion exchange resins. EP-A-0,054,152 discloses a process for the manufacture of alcohols by hydrogenation of alkyl esters of carboxylic acids. In order to remove from the products nitrogen compounds present because of the use of nitrogen-containing promotors in the manufacture of the esters, the hydrogenation products are subjected to treatment with an acidic ion exchanger. One suitable exchanger disclosed is a copolymer of acrylic acid, or a derivative thereof and divinylbenzene carrying carboxyl groups. However, it has been found that these treatments have drawbacks, for example the occurrence of side reactions, particularly in those instances where the hydrocarbon or alcohol is susceptible to acid-catalysed reactions. Such side reactions may occur because many relatively pure hydrocarbons will react when exposed to strong acid, for example olefins will react to form dimers, trimers and higher polymers. Also, alcohols will dehydrate to water, and olefins or ethers.

Such sensitivity to acid-catalyzed reactions is especially important in instances such as the etherification or alcoholification of olefins, where the starting materials may be combined prior to their reaction in the presence of a catalyst. A method for treatment of the starting materials to remove catalyst-poisoning impurities, which does not itself cause detrimental reactions in the starting materials, would be highly desirable.

It has been discovered that cationic and ammoniacal impurities can be efficiently removed from a feed stream comprising at least one alcohol and at least one olefin-containing $C_4$—$C_6$ liquid hydrocarbon, by contact with a weak acid cation exchange resin in the hydrogen form. The cation exchange resins are selected on the basis of their inability to promote acid-catalyzed reactions in the alcohol and hydrocarbon feed.

In the production of ethers it is necessary to remove cationic and ammoniacal impurities because they are catalyst poisons. However, the removal of impurities for any reason, while avoiding acid-catalyzed reaction of the materials being treated, is within the scope of the invention.

The reaction of isobutylene and methanol to form methyl tertiary-butyl ether (MTBE), a gasoline additive, is particularly well-suited to describe the invention. Other olefins such as isopentane and isohexane can also be used with alcohol reactants to form a variety of ethers.

Water can be substituted for the alcohol reactant and will form an alcohol product upon reaction with the olefin. Tertiary-butyl alcohol, also a gasoline additive, is a common application of the addition of water to an olefin.

A typical process for the preparation of MTBE uses an isobutylene (isobutene) containing hydrocarbon and methanol as the starting materials. Pure isobutylene may of course be used, but hydrocarbon mixtures containing 10 to 50 percent isobutylene are also useful. Such mixtures may contain, in addition to isobutylene, n-butane, isobutane, butene-1 and butene-2. For example, the $C_4$ fractions which are obtained by thermal cracking, steam cracking and catalytic cracking can be conveniently used. Commercially available methanol is satisfactory for use in the process.

The reactants are introduced in separate or combined streams into a suitable reactor, typically of the fixed bed shell and tube type, or a fluidized bed. Methanol is typically fed in stoichiometric excess. In passing through the catalyst bed of strongly acid cation exchange resin, the isobutylene and methanol are heated by the strongly exothermic reaction. The $C_4$ hydrocarbons other than isobutylene pass through the catalyst bed unreacted. The product MTBE and unreacted methanol and $C_4$ hydrocarbons are separated downstream of the reactor by distillation.

Examples of strong acid ion exchange resins used as the catalyst bed for MTBE synthesis include Lewatit SPC 118, Amberlyst 15, Dowex MSC-1, Duolite C-26H and Duolite ES-276. Clearly, if cationic impurities are present in the feed stream to the catalyst, they will bind to the acid sites of the catalyst and render them inactive. Further, ammonia in the feed will form the ammonium ion in the acidic environment and behave similarly.

While strong acid ion exchange resins in the hydrogen form may be used to purify methanol prior to its introduction into the reactor, such resins cannot be used to clean the $C_4$ stream as isobutylene is extremely susceptible to dimerization and polymerization under these conditions. The isobutylene thus polymerised is not available as a reactant and, further, the polymer acts as a catalyst poison, presumably by accumulating in the pores of the catalyst in the reactor. Strong acid ion exchange resins not in the hydrogen form (e.g. sodium form) are not suitable for $C_4$ stream purification since the sodium ions eluted would also poison the reactor catalyst.

2

While acid and water washes are an acceptable method of cleaning the $C_4$ feed stream to an MTBE reactor, this procedure cannot be used for purifying the methanol feed stream due to its miscibility with water. Further, it is not feasible to combine the two feed streams and purify them simultaneously using a strong acid ion exchange resin. Under these conditions the formation of MTBE will be catalyzed by the ion exchange resin. Using existing technology two cleaning techniques are required to remove impurities from the feed to an MTBE reactor, one for the isobutylene-containing $C_4$ stream and one for the methanol stream.

Typical impurities found in the $C_4$ stream include basic compounds such as ammonia and amines. Commercial methanol also generally contains traces of ammoniacal compounds as well as cationic impurities, usually cations of metals such as Cu, Fe, Ni and Cr. It has unexpectedly been found that such reactants can be combined and the impurities efficiently and economically removed by contacting the reactants, in accordance with the present invention, with a weak acid cation exchange resin which does not promote acid-catalyzed reactions in the combined feed. Such resins can be generally characterized by their ability to be converted or regenerated to the hydrogen form by contact with a nearly stoichiometric quantity of acid. This characteristic contrasts with that of the strong acid cation exchange resins, which require a large excess (usually 4 to 5 times) of strong acid in high concentration to achieve complete conversion of the resin to hydrogen form. A preferred acid cation exchange resin having the required properties has carboxylic functional groups, and may be in macroporous form.

The invention is more fully described in the following specific examples.

## Example 1

A mixture of $C_4$ aliphatic hydrocarbon containing 45 percent isobutylene, 35 percent isobutane and 20 percent 1-butene was obtained and 1.18 kg (2.6 pounds) were weighed into a 3.79 l (one gallon), stirred, isothermal autoclave. The $C_4$ mixture was spiked with 40 ppm ammonia. A measured volume of liquid $C_4$ was removed and expanded to a gas in a sealed vial with a known quantity of 0.01N sulphuric acid, then shaken. The ammonia content of the $C_4$ thus reacted with the acid to form ammonium sulphate, and the resulting solution was analysed by ion exchange chromatography to determine the initial ammonium ion concentration. About 10 g of a macroporous carboxylic acid cation exchange resin in the hydrogen form (Duolite C-464) was injected into the autoclave containing the spiked liquid $C_4$ mixture, under a nitrogen blanket. After one hour of stirring at 50 deg C a sample was removed, acidified and analysed as described above. The analysis indicated that the ammonia had been completely removed from the $C_4$ mixture.

## Example 2

The cation exchange resin described in Example 1 was evaluated for its ability to remove copper ions from methanol. Methanol was spiked with 300 ppm $Cu^{2+}$ derived from cupric sulfate. The methanol was then passed through a 26 ml vertical column filled with the cation exchange resin at a rate of 30 bed volumes per hour at room temperature. Cupric ion concentration in the effluent was monitored by a visible spectrophotometer. The resin effectively removed copper from the methanol, with initial breakthrough from the column occurring at 3 bed volumes.

## Example 3

The resin described in Example 1 was evaluated for its catalytic effect on the formation of isobutylene dimers. About 1.18 kg (2.6 pounds) of the $C_4$ mixture of Example 1 was weighed into the 3.79 l (one gallon) autoclave reactor. The reactor temperature was raised to 70 deg C and at time zero the dry equivalent of 120 ml of wet resin was injected into the reactor. Samples were taken periodically and analysed by gas chromatography for isobutylene dimer.

In order to investigate the catalytic effect of the resin, the data of percent conversion versus time was fitted to a rate law. A first order rate law of the following form was assumed:

$$r = kC_{IB} = kC_o(1 - X)$$

where r is the rate of consumption of isobutylene in g moles/l of resin per hour. $C_{IB}$ is the concentration of isobutylene, $C_o$ is the initial isobutylene concentration, X is the fraction of isobutylene reacted and k is the rate constant with units of hour$_{-1}$.

The rate constant for isobutylene dimer formation at 70 deg C is shown in Table I.

TABLE I

| Resin (hydrogen form) | Rate Constant |
|---|---|
| Carboxylic acid cation exchange (Duolite C-464) | 0.00 |

The resin did not have a catalytic effect on the formation of isobutylene dimer.

Example 4

Typical feed streams of olefin and methanol for the preparation of MTBE were combined and treated by the process of the invention to demonstrate that the treatment does not catalyze the MTBE reaction. A 3.79 1 (one gallon) autoclave reactor was charged with 460 ml of methanol and 1.18 kg (2.6 pounds) of the $C_4$ mixture described in Example 1. At time zero the dry equivalent of 120 ml of wet resin expanded in water was injected into the reactor with nitrogen. Samples were taken periodically and analysed by gas chromatography for isobutylene content. The percent isobutylene conversion was plotted against time, and the data was fitted to a rate law of the following form:

$$r = k_{forward}C_{IB} - k_{reverse}C_{MTBE} = kC_o(1 - X - KX)$$

where K is an equilibrium constant equal to 0.0526 at 70 deg C.

Table II lists the rate constant for the MTBE reaction in the presence of a cation exchange resin useful in the invention, and also in the presence of a strong acid macroporous cation exchange resin known to be a good catalyst for MTBE formation.

TABLE II

| Resin (hydrogen form) | Rate Constant |
|---|---|
| Carboxylic acid cation exchange (Duolite C-464) | 0.0 |
| Sulphonated strong acid cation exchange (Duolite ES-276) | 3.1 |

The quantity of MTBE produced by contact with resin useful in the process of the invention is not significant. Impurities can be removed using this resin on a combined feed stream without generating sufficient MTBE to require additional heat removal equipment of the exothermic reaction.

"Lewatit", "Amberlyst", "Duolite" and "Dowex" are trademarks which may be registered in one or more of the Designated States.

**Claims**

1. A method for the removal of cationic and ammoniacal impurities from a feed stream comprising at least one alcohol and at least one olefin-containing $C_4$—$C_6$ liquid hydrocarbon, the impurities being removed by contacting the feed stream with a weak acid cation exchange resin in the hydrogen form.

2. A method according to claim 1, wherein the feed stream contains methanol.

3. A method according to claim 2, wherein the feed stream is methanol and isobutylene.

4. A method according to any preceding claim, wherein the cationic impurities are metal ions.

5. A method according to any preceding claim, wherein the resin contains carboxylic acid functional groups.

6. A method for the production of methyl tertiary-butyl ether by the reaction of isobutylene and methanol in the presence of an ion exchange catalyst, characterised in that isobutylene and methanol feed streams are combined and the combined stream is contacted, prior to undergoing the reaction, with a weak acid cation exchange resin in the hydrogen form.

**Patentansprüche**

1. Verfahren zum Entfernen kationischer und ammoniakalischer Verunreinigungen aus einem Beschickungsstrom, enthaltend mindestens einen Alkohol und mindestens einen olefinhaltigen $C_4$—$C_6$ flüssigen Kohlenwasserstoff, wobei man die Verunreinigungen entfernt durch Kontaktieren des Beschickungsstroms mit einem schwach sauren Kationenaustauscherharz in der Wasserstofform.

2. Verfahren nach Anspruch 1, wobei der Beschickungsstrom Methanol enthält.

3. Verfahren nach Anspruch 2, wobei der Beschickungsstrom Methanol und Isobutylen ist.

4. Verfahren nach einem der vorhergehenden Ansprüche, wobei die kationischen Verunreinigungen Metallionen sind.

5. Verfahren nach einem der vorhergehenden Ansprüche, wobei das Harz funktionelle Carbonsäuregruppen enthält.

6. Verfahren zur Herstellung von Methyl-t-butylether durch Reaktion von Isobutylen und Methanol in Gegenwart eines Ionenaustauscher-Katalysators, dadurch gekennzeichnet, daß man Isobutylen- und Methanolbeschickungsströme kombiniert und die kombinierten Ströme, bevor sie der Reaktion unterzogen werden, mit einem schwach sauren Kationenaustauscherharz in der Wasserstofform in Kontakt bringt.

# EP 0 125 833 B1

**Revendications**

1. Procédé pour enlever les impuretés cationiques et ammoniacales à partir d'un courant d'alimentation comprenant au moins un alcool et au moins un hydrocarbure liquide en C$_4$—C$_6$ contenant de l'oléfine, les impuretés étant éliminées par mise en contact du courant d'alimentation avec une résine échangeuse de cations faiblement acide sous la forme hydrogène.

2. Procédé selon la revendication 1, dans lequel le courant d'alimentation contient du méthanol.

3. Procédé selon la revendication 2, dans lequel le courant d'alimentation est du méthanol et de l'isobutylène.

4. Procédé selon l'une quelconque des revendications précédentes, dans lequel les impuretés cationiques sont des ions métalliques.

5. Procédé selon l'une quelconque des revendications précédentes, dans lequel la résine contient des groupes fonctionnels acide carboxylique.

6. Procédé pour la production de méthyltertiobutyléther par réaction de l'isobutylène et du méthanol en présence d'un catalyseur échangeur d'ions, caractérisé en ce que les courants d'alimentation d'isobutylène et de méthanol sont combinés et en ce que le courant combiné est mis en contact, avant de subir la réaction, avec une résine échangeuse de cations faiblement acide sous la forme hydrogène.

5